# EUROPEAN PATENT APPLICATION

(11) **EP 4 672 040 A1**
(43) Date of publication of application: **31.12.2025**
(21) Application number: 24760331.9
(22) Date of filing: 20.02.2024
(51) Int. Cl.: G06F 18/15, G06N 5/02, G06N 20/00, G16H 10/00, G16H 50/00

(54) **DISEASE PREDICTION DEVICE, LEARNING MODEL GENERATION DEVICE, DISEASE PREDICTION METHOD, LEARNING MODEL GENERATION METHOD, AND COMPUTER-READABLE RECORDING MEDIUM**

(30) Priority: 22.02.2023 JP 2023026656
(71) Applicant: NEC Solution Innovators, Ltd., Koto-ku, Tokyo 136-8627 (JP); Tohoku University, Sendai-shi, Miyagi 980-8577 (JP); NEC Corporation, 108-8001 Tokyo (JP)
(72) Inventor: KATO, Shintaro, Tokyo 136-8627 (JP); HORII, Katsunori, Tokyo 136-8627 (JP); KANEKO, Naoto, Tokyo 136-8627 (JP); YASUDA, Satoshi, Sendai-shi Miyagi 980-8577 (JP); SHIMOKAWA, Hiroaki, Sendai-shi Miyagi 980-8577 (JP); NOCHIOKA, Kotaro, Sendai-shi Miyagi 980-8577 (JP); PILEGGI, Giampaolo, 69115 Heidelberg (DE)
(74) Representative: Betten & Resch
(86) International application number: PCT/JP2024/005904
(87) International publication number: WO 2024/177033

(57) **Abstract**

A learning model generation apparatus 10 comprises: a graph generation unit 11 which generates, from a data group including biometric information of persons and information indicating the presence or absence of occurrence of diseases in the persons, a graph composed of nodes representing data points and edges representing relationships between the nodes; a graph supplementation unit 12 which supplements the generated graph for a deficiency therein; and a model generation unit 13 which generates, from the supplemented graph, a data group in which the deficiency is supplemented, performs machine learning using the generated data group as training data, and generates a prediction model for predicting the occurrence of diseases in a person.

## Description

### Technical Field

The present disclosure relates to a disease prediction apparatus and a disease prediction method for supporting diagnosis of a disease by a doctor, and further relates to a computer-readable recording medium in which a program for achieving the disease prediction apparatus and the disease prediction method is recorded. The present disclosure relates also to a learning model generation apparatus and a learning model generation method for generating a learning model used in the above-described disease prediction apparatus, and further relates to a computer-readable recording medium recording a program for achieving the learning model generation apparatus and the learning model generation method.

### Background Art

In order to maintain a healthy life of a person, it is important to predict the occurrence of a serious disease that is life threatening, such as heart failure, renal failure, or cerebral infarction. For this reason, for example, PTL 1 discloses a system that predicts a future disease of a person using a prediction model.

In the system disclosed in PTL 1, the prediction model is a machine learning model. The prediction model is constructed by machine learning using, as training data, human health data or vital data (explanatory variable) and presence or absence of onset of a disease (objective variable) serving as teacher data. Examples of the health data include data obtained by a test such as a blood test, such as weight, height, CCR (creatinine clearance), and cholesterol level. Examples of the vital data include data obtained from a living body via a sensor, such as a blood pressure, a heart rate, a respiratory rate, a body temperature, a wake-up time, a bed time, and a sleep time.

Therefore, the system disclosed in PTL 1 acquires health data or vital data of a patient to be predicted, and inputs the acquired data to a machine learning model. Then, the system disclosed in PTL 1 displays the output of the machine learning model as a prediction result. The doctor can make a final diagnosis while checking the prediction result.

### Citation List

### Patent Literature

PTL 1: JP 2022-169193 A

### Summary of Invention

### Technical Problem

Meanwhile, in the system disclosed in PTL 1, it is necessary to prepare as much training data as possible and execute machine learning in order to improve prediction accuracy. However, the human health data and the vital data serving as the training data include different types of values depending on the person at the collection destination, and are not uniform. That is, although an item a is deficient in the training data obtained from Mr. A, an item b is deficient instead of the item a in the training data obtained from Mr. B. Therefore, in the system disclosed in PTL 1, it is difficult to use a prediction model with high prediction accuracy, and it is difficult to improve the prediction accuracy.

An example of an object of the present disclosure is to improve prediction accuracy of a disease even when a deficiency exists in training data of a prediction model for predicting a disease.

### Solution to Problem

In order to achieve the above object, a learning model generation apparatus according to an aspect of the present disclosure includes
a graph generation unit for generating, from a data group including biometric information of a person and information indicating presence or absence of occurrence of a disease in the person, a graph including nodes representing data points and edges representing a relationship between the nodes,
a graph supplementation unit for supplementing a deficiency of the generated graph, and
a model generation unit for generating, from the supplemented graph, a data group in which a deficiency is supplemented, performing machine learning using the generated data group as training data, and generating a prediction model for predicting occurrence of a disease in the person.

In order to achieve the above object, a disease prediction apparatus according to an aspect of the present disclosure includes
an information acquisition unit for acquiring biometric information of a person to be predicted, and
a disease prediction unit for inputting the acquired biometric information to a prediction model and predicting presence or absence of occurrence of a disease in the person to be predicted from an output result of the prediction model,
in which the prediction model is generated by
generating, from a data group including biometric information of a person and information indicating presence or absence of occurrence of a disease in the person, a graph including nodes representing data points and edges representing a relationship between nodes
supplementing a deficiency of the generated graph,
generating a data group in which a deficiency is supplemented from the supplemented graph, and
performing machine learning using the generated data group as training data.

In order to achieve the above object, a learning model generation method according to an aspect of the present disclosure includes
a graph generation step for generating, from a data group including biometric information of a person and information indicating presence or absence of occurrence of a disease in the person, a graph including nodes representing data points and edges representing a relationship between the nodes,
a graph supplementation step for supplementing a deficiency of the generated graph, and
a model generation step for generating, from the supplemented graph, a data group in which a deficiency is supplemented, performing machine learning using the generated data group as training data, and generating a prediction model for predicting occurrence of a disease in the person.

In order to achieve the above object, a disease prediction method according to an aspect of the present disclosure includes
an information acquisition step for acquiring biometric information of a person to be predicted, and
a disease prediction step for inputting the acquired biometric information to a prediction model and predicting presence or absence of occurrence of a disease in the person to be predicted from an output result of the prediction model,
in which the prediction model is generated by
generating, from a data group including biometric information of a person and information indicating presence or absence of occurrence of a disease in the person, a graph including nodes representing data points and edges representing a relationship between nodes,
supplementing a deficiency of the generated graph,
generating a data group in which a deficiency is supplemented from the supplemented graph, and
executing machine learning using the generated data group as training data.

Furthermore, in order to achieve the above object, a first computer-readable recording medium according to an aspect of the present disclosure has recorded a program therein including commands for causing a computer to execute
a graph generation step for generating, from a data group including biometric information of a person and information indicating presence or absence of occurrence of a disease in the person, a graph including nodes representing data points and edges representing a relationship between the nodes,
a graph supplementation step for supplementing a deficiency of the generated graph, and
a model generation step for generating, from the supplemented graph, a data group in which a deficiency is supplemented, performing machine learning using the generated data group as training data, and generating a prediction model for predicting occurrence of a disease in the person.

Furthermore, in order to achieve the above object, a second computer-readable recording medium according to an aspect of the present disclosure has recorded a program therein including commands for causing a computer to execute
an information acquisition step for acquiring biometric information of a person to be predicted, and
a disease prediction step for inputting the acquired biometric information to a prediction model and predicting presence or absence of occurrence of a disease in the person to be predicted from an output result of the prediction model,
in which the prediction model is generated by
generating, from a data group including biometric information of a person and information indicating presence or absence of occurrence of a disease in the person, a graph including nodes representing data points and edges representing a relationship between nodes,
supplementing a deficiency of the generated graph,
generating a data group in which a deficiency is supplemented from the supplemented graph, and
performing machine learning using the generated data group as training data. Advantageous Effects of Invention

As described above, according to the present disclosure, even in a case where a deficiency exists in training data of a prediction model for predicting a disease, prediction accuracy of the disease can be improved.

### Brief Description of Drawings

[Fig. 1] Fig. 1 is a configuration diagram illustrating a schematic configuration of an example of a learning model generation apparatus.
[Fig. 2] Fig. 2 is a configuration diagram more specifically illustrating a configuration of an example of the learning model generation apparatus.
[Fig. 3] Fig. 3 is a diagram illustrating an example of a data group used to generate a graph.
[Fig. 4] Fig. 4 is a diagram illustrating an example of a generated graph.
[Fig. 5] Fig. 5 is a diagram illustrating an example of a data group in which a deficiency is supplemented.
[Fig. 6] Fig. 6 is a flowchart illustrating an example of an operation of the learning model generation apparatus.
[Fig. 7] Fig. 7 is a configuration diagram a configuration of an example of a disease prediction apparatus.
[Fig. 8] Fig. 8 is a flowchart illustrating an example of an operation of the disease prediction apparatus.
[Fig. 9] Fig. 9 is a block diagram illustrating an example of a computer that implements a learning model generation apparatus and a disease prediction apparatus.

### Example Embodiment

### (First example embodiment)

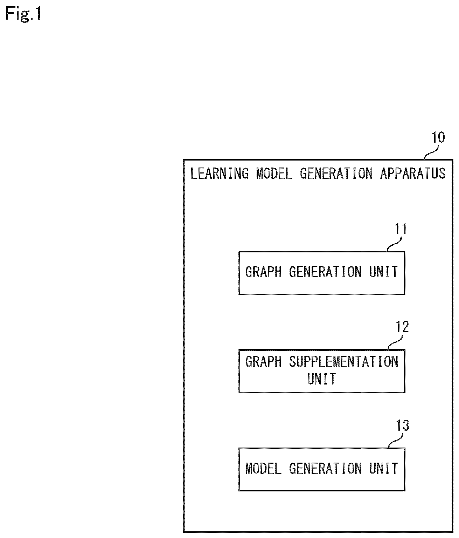

Hereinafter, in a first example embodiment, a learning model generation apparatus, a learning model generation method, and a program will be described with reference to Figs. 1 to 6.

### [Apparatus configuration]

First, a schematic configuration of an example of a learning model generation apparatus will be described with reference to Fig. 1. Fig. 1 is a configuration diagram illustrating a schematic configuration of an example of a learning model generation apparatus according to the first example embodiment.

A learning model generation apparatus 10 according to the first example embodiment illustrated in Fig. 1 is a apparatus for generating a learning model for disease prediction. As illustrated in Fig. 1, the learning model generation apparatus 10 includes a graph generation unit 11, a graph supplementation unit 12, and a model generation unit 13.

The graph generation unit 11 generates a graph from a data group including biometric information of a person and information indicating the presence or absence of occurrence of a disease in the person. The graph includes nodes representing data points and edges representing relationships between the nodes. The graph supplementation unit 12 supplements the deficiency of the graph generated by the graph generation unit 11.

The model generation unit 13 first generates a data group in which a deficiency is supplemented from the graph supplemented by the graph supplementation unit 12. Then, the model generation unit 13 executes machine learning using the generated data group as training data, and generates a prediction model 20 that predicts the occurrence of a human disease.

In this manner, the learning model generation apparatus 10 supplements a deficiency of data to be training data of the learning model by graph supplementation. Then, the deficiency is supplemented with high accuracy by graph supplementation. Therefore, according to the learning model generation apparatus 10, even in a case where there is a variation in training data of a prediction model for predicting a disease, the prediction accuracy of a disease is improved.

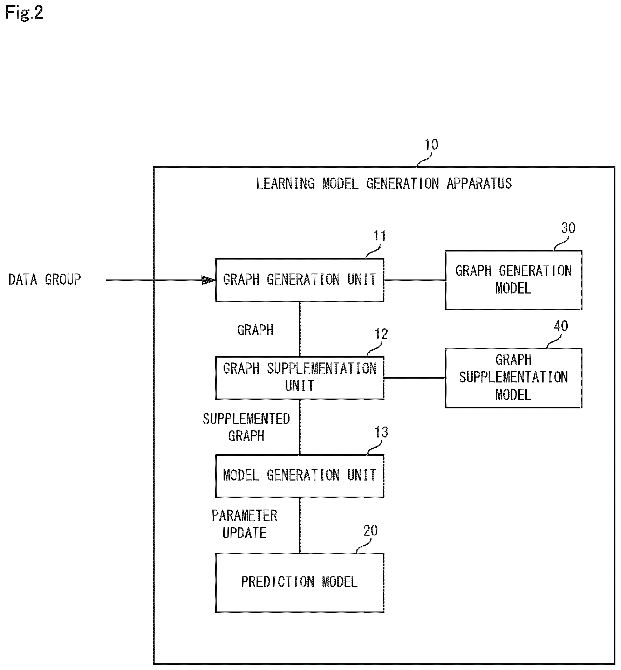

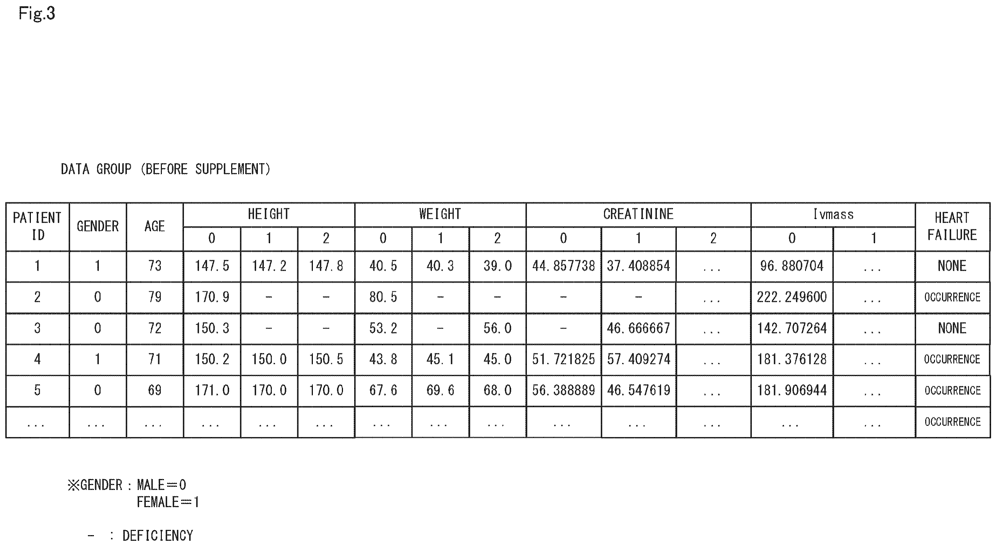

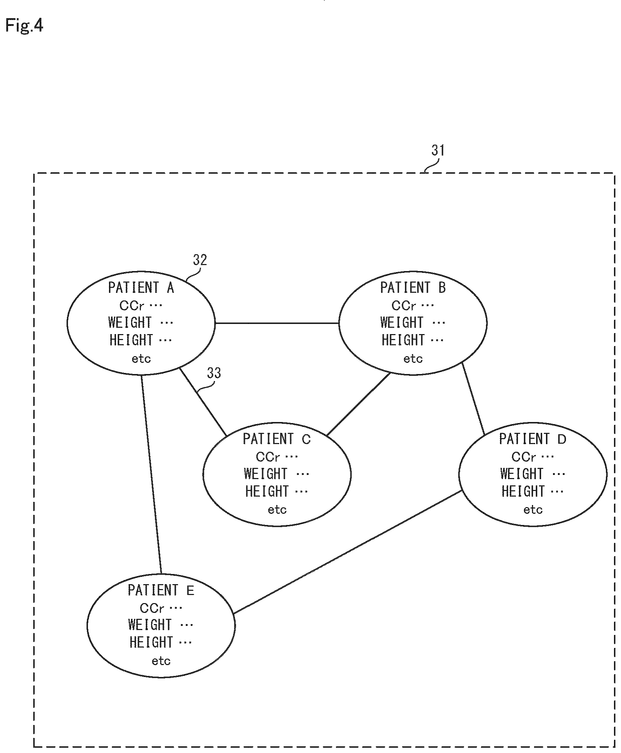

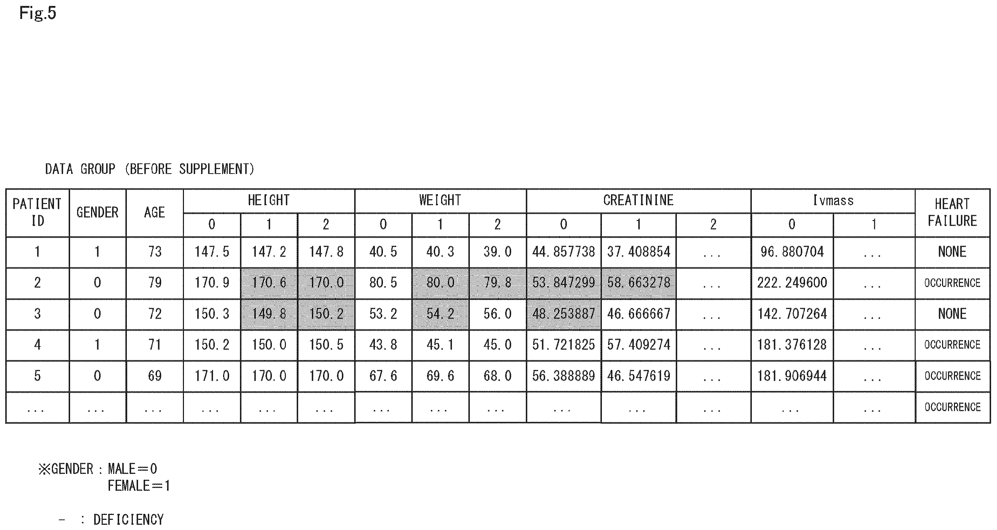

Next, a configuration and a function of the learning model generation apparatus will be specifically described with reference to Figs. 2 to 5. Fig. 2 is a configuration diagram more specifically illustrating a configuration of an example of the learning model generation apparatus. Fig. 3 is a diagram illustrating an example of a data group used to generate a graph. Fig. 4 is a diagram illustrating an example of a generated graph. Fig. 5 is a diagram illustrating an example of a data group in which a deficiency is supplemented.

First, as illustrated in Fig. 2, the learning model generation apparatus 10 includes a prediction model 20, a graph generation model 30, and a graph supplementation model 40 in addition to the graph generation unit 11, the graph supplementation unit 12, and the model generation unit 13 described above.

The prediction model 20, the graph generation model 30, and the graph supplementation model 40 are machine learning models. Examples of the machine learning model include a neural network. The prediction model 20, the graph generation model 30, and the graph supplementation model 40 are actually implemented by a machine learning program executed on a computer. These machine learning models may be constructed outside the learning model generation apparatus 10. Details of these machine learning models will be described later.

In the first example embodiment, as described above, the data group includes biometric information of a person and information indicating the presence or absence of occurrence of a disease in the person. In the first example embodiment, examples of the biometric information include gender, age, height, weight, blood pressure, creatinine clearance (CCr), Ivmass, cholesterol level, and the like, and the biometric information may be time-series information acquired along a time series. Examples of the disease include heart failure, renal failure, cerebral infarction, and diabetes.

In the example illustrated in Fig. 3, the data group includes gender, age, height, weight, creatinine clearance (CCr), and Ivmass for each patient (for each ID) as the biometric information. The data group also includes information indicating the presence or absence of occurrence of heart failure for each patient. The information indicating the presence or absence of occurrence of a disease may include the presence or absence of occurrence of a disease for each elapsed time.

In the first example embodiment, the graph generation unit 11 first acquires the data group illustrated in Fig. 3 from an external server apparatus or the like. As illustrated in Fig. 3, the acquired data group has a deficiency. Therefore, in a case where the prediction model is generated using the data group as it is, it is difficult to increase the accuracy of the prediction model.

Subsequently, the graph generation unit 11 generates a graph 31 (see Fig. 4) from the data group in which the deficiency exists. Fig. 4 conceptually illustrates the generated graph. As illustrated in Fig. 4, the graph 31 includes a set of a large number of nodes 32 and a set of edges 33 connecting the nodes. The node 32 represents an entity.

As illustrated in Fig. 4, in the graph 31, the edge 33 connects the nodes 32 of similar entities. For example, in the example of Fig. 4, since patient A and patient B have similar attributes, a node representing patient A and a node representing patient B are connected by the edge 33. Each node 32 includes biometric information of the relevant patient.

In the first example embodiment, the graph generation unit 11 generates a graph by inputting the acquired data group to the graph generation model 30. The graph generation model 30 performs machine learning of a relationship between nodes to be connected by edges.

In the construction of the graph generation model 30, a combination of entities and a label indicating whether the combination should be connected are used as training data. The following document is referred to for the construction of the graph generation model 30.

Reference Document: NEC Technical Journal/Vol. 72 No. 1/AI Special issue that creates new social value 103, October 2019

In the first example embodiment, the graph supplementation unit 12 supplements the deficiency of the graph 31 generated by the graph generation unit 11 using the graph supplementation model 40. The graph supplementation model 40 is a machine learning model constructed by performing machine learning on a relationship between a graph in which some nodes are deficient and a graph in which the deficiency is supplemented.

In the construction of the graph supplementation model 40, the feature amount representing the graph in which the node is deficient and the feature amount of the graph in which the deficiency is supplemented are obtained, and the obtained two feature amounts are used as training data. The above document is also referred to for the graph supplementation model 40.

When the deficiency of the graph is supplemented by the graph supplementation unit 12, the model generation unit 13 converts the graph in which the deficiency is supplemented into the original data group. Specifically, the model generation unit 13 specifies a node relevant to the patient, and specifies a node other than the patient connected to the specified node. Then, the model generation unit 13 combines the entities of the specified nodes into one record.

By the conversion processing described above, as illustrated in Fig. 5, the deficient part of the data group is supplemented. Fig. 5 is an example in which a deficient part of the data group illustrated in Fig. 2 is supplemented. In Fig. 5, a gray part is a supplemented part.

Subsequently, the model generation unit 13 inputs the supplemented data group illustrated in Fig. 5 to the prediction model 20 for each record, and updates the parameter of the prediction model 20 so that the output result matches the presence or absence of occurrence of a disease in the data group. In the first example embodiment, the prediction model 20 is generated by updating the parameters of the prediction model 20. That is, in the first example embodiment, the generation of the prediction model includes updating the parameter.

### [Apparatus operation]

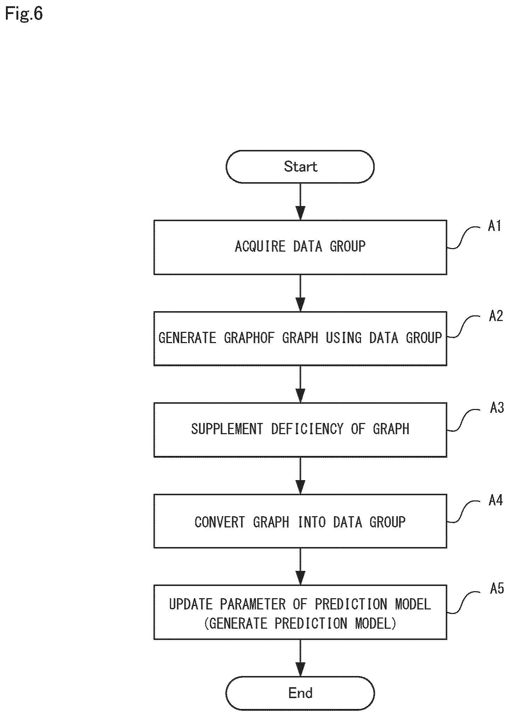

Next, an example of the operation of the learning model generation apparatus 10 will be described with reference to Fig. 6. Fig. 6 is a flowchart illustrating an example of the operation of the learning model generation apparatus. In the following description, Figs. 1 to 5 will be appropriately referred to. In the first example embodiment, the learning model generation method is performed by operating the learning model generation apparatus 10. Therefore, the description of the learning model generation method in the first example embodiment is replaced with the following description of the operation of the learning model generation apparatus 10.

As illustrated in Fig. 6, first, the graph generation unit 11 acquires a data group (see Fig. 3) including biometric information of a person and information indicating the presence or absence of occurrence of a disease in the person from an external server apparatus or the like (step A1). The acquired data group has a deficiency.

Next, the graph generation unit 11 inputs the data group in which the deficiency exists to the graph generation model 30 to generate the graph 31 (see Fig. 4) (step A2). Since the deficiency exists in the data group, the deficiency also exists in the graph 31.

Next, the graph supplementation unit 12 supplements the deficiency of the graph 31 generated by the graph generation unit 11 in step A2 using the graph supplementation model 40 (step A3). Specifically, the graph supplementation unit 12 inputs the graph 31 to the graph supplementation model 40 to supplement the deficiency of the graph 31.

Next, when the deficiency of the graph is supplemented in step A3, the model generation unit 13 converts the graph in which the deficiency is supplemented into the original data group (step A4). In the data group obtained in step A4, the deficiency is supplemented (see Fig. 5).

Next, the model generation unit 13 inputs the data group obtained in step A4 to the prediction model 20 for each record, and updates the parameters of the prediction model 20 so that the output result matches the presence or absence of occurrence of a disease in the data group (step A5). By updating the parameter in step A5, the prediction model 20 is finally generated.

As described above, in the first example embodiment, the deficiency of the data group serving as the training data is supplemented by graph supplementation. Then, the prediction model is constructed by the data group in which the deficiency is supplemented. Since the supplementation is performed by graph supplementation, it can be said that the accuracy of the supplementation is high. Therefore, according to the first example embodiment, it is possible to improve the prediction accuracy of a disease even when there is a variation in the training data of the prediction model for predicting the disease.

### [Program]

The program in the first example embodiment may be a program that causes a computer to execute steps A1 to A5 illustrated in Fig. 6. By installing and executing this program in the computer, the learning model generation apparatus 10 and the learning model generation method according to the first example embodiment can be achieved. In this case, the processor of the computer functions as the graph generation unit 11, the graph supplementation unit 12, and the model generation unit 13, and performs processing. Examples of the computer include a smartphone and a tablet terminal apparatus in addition to a general-purpose PC.

The program according to the first example embodiment may be executed by a computer system constructed by a plurality of computers. In this case, for example, each computer may function as any of the graph generation unit 11, the graph supplementation unit 12, and the model generation unit 13.

### (Second example embodiment)

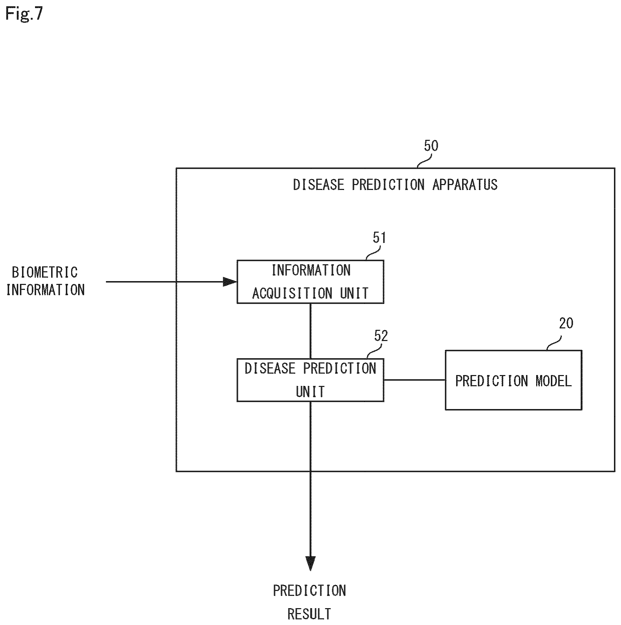

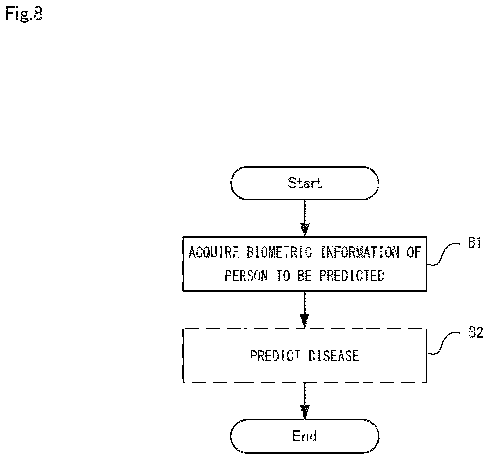

Next, a disease prediction apparatus, a disease prediction method, and a program in a second example embodiment will be described with reference to Figs. 7 and 8.

### [Apparatus configuration]

First, a configuration of an example of a disease prediction apparatus will be described with reference to Fig. 7. Fig. 7 is a configuration diagram illustrating a configuration of an example of a disease prediction apparatus.

A disease prediction apparatus 50 according to the second example embodiment illustrated in Fig. 7 is an apparatus for predicting a disease of a person. As illustrated in Fig. 7, the disease prediction apparatus 50 includes an information acquisition unit 51 and a disease prediction unit 52.

The information acquisition unit 51 acquires biometric information of a person to be predicted from an external server apparatus or the like. As in the first example embodiment, examples of the biometric information include gender, age, height, weight, blood pressure, creatinine clearance (CCr), Ivmass, cholesterol level, and the like.

The disease prediction unit 52 inputs the biometric information acquired by the information acquisition unit 51 to the prediction model 20, and predicts the presence or absence of occurrence of a disease in a person to be predicted from the output result of the prediction model 20.

The prediction model 20 is generated by the following steps (a) to (d).
(a) A step of generating a graph including nodes representing data points and edges representing a relationship between nodes from a data group including biometric information of a person and information indicating presence or absence of occurrence of a disease in the person.
(b) A step of supplementing the deficiency of the generated graph.
(c) A step of generating a data group in which a deficiency is supplemented from the supplemented graph.
(d) A step of executing machine learning using the generated data group as training data.

That is, the prediction model 20 is the same as the prediction model 20 described in the first example embodiment. When biometric information of a person to be predicted is input, the disease prediction unit 52 predicts a disease of a person by inputting the biometric information to the prediction model 20 described in the first example embodiment.

### [Apparatus operation]

Next, an example of the operation of the disease prediction apparatus 50 will be described with reference to Fig. 8. Fig. 8 is a flowchart illustrating an example of the operation of the disease prediction apparatus. In the following description, Fig. 7 is appropriately referred to. In the second example embodiment, the disease prediction method is performed by operating the disease prediction apparatus 50. Therefore, the description of the disease prediction method in the second example embodiment is replaced with the following description of the operation of the disease prediction apparatus 50.

As illustrated in Fig. 8, first, the information acquisition unit 51 acquires biometric information of a person to be predicted from an external server apparatus or the like (step B1).

Next, the disease prediction unit 52 inputs the biometric information acquired by the information acquisition unit 51 in Step B1 to the prediction model 20, and predicts the presence or absence of occurrence of a disease in a person to be predicted from the output result of the prediction model 20 (Step B2).

Specifically, in step B2, the disease prediction unit 52 transmits the output result of the prediction model 20 to a terminal apparatus such as a doctor who has requested prediction. As a result, the doctor or the like confirms the prediction result of the disease of the person (patient) to be predicted.

As described above, in the second example embodiment, since the prediction model constructed in the first example embodiment is used, a highly accurate prediction result of a disease can be obtained.

### [Program]

The program in the second example embodiment may be any program that causes a computer to execute steps B1 and B2 illustrated in Fig. 8. By installing and executing this program in a computer, the disease prediction apparatus 50 and the disease prediction method according to the second example embodiment can be achieved. In this case, the processor of the computer functions as the information acquisition unit 51 and the disease prediction unit 52, and performs processing. Examples of the computer include a smartphone and a tablet terminal apparatus in addition to a general-purpose PC.

The program according to the second example embodiment may be executed by a computer system constructed by a plurality of computers. In this case, for example, each computer may function as the information acquisition unit 51 or the disease prediction unit 52.

### [Physical configuration]

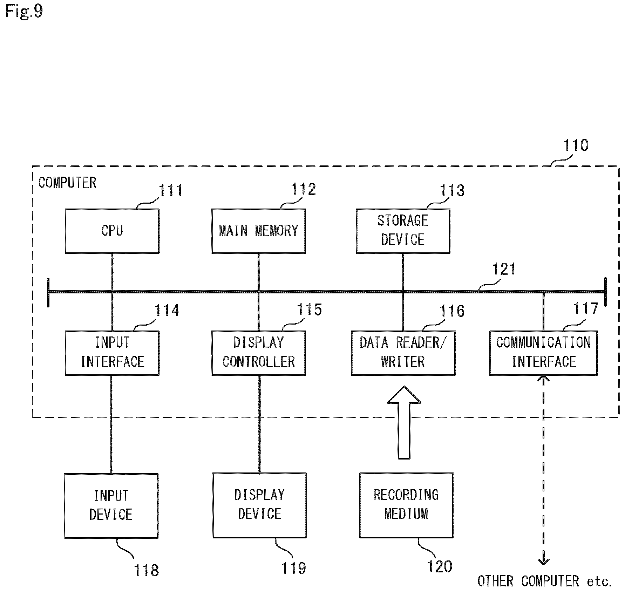

Here, a computer that implements the learning model generation apparatus 10 and the disease prediction apparatus 50 by executing a program will be described with reference to Fig. 9. Fig. 9 is a block diagram illustrating an example of a computer that implements the learning model generation apparatus and the disease prediction apparatus.

As illustrated in Fig. 9, a computer 110 includes a central processing unit (CPU) 111, a main memory 112, a storage device 113, an input interface 114, a display controller 115, a data reader/writer 116, and a communication interface 117. These units are data-communicably connected to each other via a bus 121.

The computer 110 may include a graphics processing unit (GPU) or a field-programmable gate array (FPGA) in addition to the CPU 111 or instead of the CPU 111. In this aspect, the GPU or the FPGA can execute the program in the example embodiment.

The CPU 111 develops the program according to the example embodiment, which is stored in the storage device 113 and configured by a code group, in the main memory 112, and executes each code in a predetermined order to perform various operations. The main memory 112 is typically a volatile storage device such as a dynamic random access memory (DRAM).

The program according to the example embodiment is provided in a state of being stored in the computer-readable recording medium 120. The program in the present example embodiment may be distributed on the Internet connected via the communication interface 117.

Specific examples of the storage device 113 include a semiconductor storage device such as a flash memory in addition to a hard disk drive. The input interface 114 mediates data transmission between the CPU 111 and the input device 118 such as a keyboard and a mouse. The display controller 115 is connected to a display device 119 and controls display on the display device 119.

The data reader/writer 116 mediates data transmission between the CPU 111 and the recording medium 120, and reads a program from the recording medium 120 and writes a processing result in the computer 110 to the recording medium 120. The communication interface 117 mediates data transmission between the CPU 111 and another computer.

Specific examples of the recording medium 120 include general-purpose semiconductor storage devices such as a CF (Compact Flash (registered trademark)) and an SD (Secure Digital), a magnetic recording medium such as a flexible disk, and an optical recording medium such as a CD-ROM (Compact Disk Read Only Memory).

The learning model generation apparatus 10 and the disease prediction apparatus 50 can also be achieved by using hardware relevant to each unit, for example, an electronic circuit, instead of a computer in which a program is installed. Furthermore, a part of the learning model generation apparatus 10 and the disease prediction apparatus 50 may be achieved by a program, and the remaining part may be achieved by hardware. The computer is not limited to the computer illustrated in Fig. 9.

Some or all of the above-described example embodiments can be expressed by (Supplementary Note 1) to (Supplementary Note 18) described below, but are not limited to the following description.

### (Supplementary Note 1)

A learning model generation apparatus including
a graph generation unit for generating, from a data group including biometric information of a person and information indicating presence or absence of occurrence of a disease in the person, a graph including nodes representing data points and edges representing a relationship between the nodes,
a graph supplementation unit for supplementing a deficiency of the generated graph, and
a model generation unit for generating, from the supplemented graph, a data group in which a deficiency is supplemented, performing machine learning using the generated data group as training data, and generating a prediction model for predicting occurrence of a disease in the person.

### (Supplementary Note 2)

The learning model generation apparatus according to Supplementary Note 1, in which
the graph supplementation unit supplements a deficiency of the generated graph using a machine learning model constructed by performing machine learning on a relationship between a graph in which some nodes are deficient and a graph in which a deficiency is supplemented.

### (Supplementary Note 3)

The learning model generation apparatus according to Supplementary Note 1, in which
the biometric information includes time-series information acquired along a time series from each of a plurality of persons.

### (Supplementary Note 4)

The learning model generation apparatus according to Supplementary Note 3, in which
information indicating presence or absence of occurrence of a disease in the person includes presence or absence of occurrence of the disease for each elapsed time.

### (Supplementary Note 5)

The learning model generation apparatus according to Supplementary Note 1, in which
the disease is heart failure, renal failure, cerebral infarction, or diabetes.

### (Supplementary Note 6)

A disease prediction apparatus including
an information acquisition unit for acquiring biometric information of a person to be predicted, and
a disease prediction unit for inputting the acquired biometric information to a prediction model and predicting presence or absence of occurrence of a disease in the person to be predicted from an output result of the prediction model,
wherein the prediction model is generated by
generating, from a data group including biometric information of a person and information indicating presence or absence of occurrence of a disease in the person, a graph including nodes representing data points and edges representing a relationship between nodes,
supplementing a deficiency of the generated graph,
generating a data group in which a deficiency is supplemented from the supplemented graph, and
performing machine learning using the generated data group as training data.

### (Supplementary Note 7)

A learning model generation method including
a graph generation step for generating, from a data group including biometric information of a person and information indicating presence or absence of occurrence of a disease in the person, a graph including nodes representing data points and edges representing a relationship between the nodes,
a graph supplementation step for supplementing a deficiency of the generated graph, and
a model generation step for generating, from the supplemented graph, a data group in which a deficiency is supplemented, performing machine learning using the generated data group as training data, and generating a prediction model for predicting occurrence of a disease in the person.

### (Supplementary Note 8)

The learning model generation method according to Supplementary Note 7, in which
the graph supplementation step includes supplementing a deficiency of the generated graph using a machine learning model constructed by performing machine learning on a relationship between a graph in which some nodes are deficient and a graph in which a deficiency is supplemented.

### (Supplementary Note 9)

The learning model generation method according to Supplementary Note 7, in which
the biometric information includes time-series information acquired along a time series from each of a plurality of persons.

### (Supplementary Note 10)

The learning model generation method according to Supplementary Note 9, in which
information indicating presence or absence of occurrence of a disease in the person includes presence or absence of occurrence of the disease for each elapsed time.

### (Supplementary Note 11)

The learning model generation method according to Supplementary Note 7, in which
the disease is heart failure, renal failure, cerebral infarction, or diabetes.

### (Supplementary Note 12)

A disease prediction method including
an information acquisition step for acquiring biometric information of a person to be predicted, and
a disease prediction step for inputting the acquired biometric information to a prediction model and predicting presence or absence of occurrence of a disease in the person to be predicted from an output result of the prediction model,
in which the prediction model is generated by
generating, from a data group including biometric information of a person and information indicating presence or absence of occurrence of a disease in the person, a graph including nodes representing data points and edges representing a relationship between nodes,
supplementing a deficiency of the generated graph,
generating a data group in which a deficiency is supplemented from the supplemented graph, and
performing machine learning using the generated data group as training data.

### (Supplementary Note 13)

A computer-readable recording medium having recorded therein a program including commands for causing a computer to execute
a graph generation step for generating, from a data group including biometric information of a person and information indicating presence or absence of occurrence of a disease in the person, a graph including nodes representing data points and edges representing a relationship between the nodes,
a graph supplementation step for supplementing a deficiency of the generated graph, and
a model generation step for generating, from the supplemented graph, a data group in which a deficiency is supplemented, performing machine learning using the generated data group as training data, and generating a prediction model for predicting occurrence of a disease in the person.

### (Supplementary Note 14)

The computer-readable recording medium according to Supplementary Note 13, in which
the graph supplementation step includes supplementing a deficiency of the generated graph using a machine learning model constructed by performing machine learning on a relationship between a graph in which some nodes are deficient and a graph in which a deficiency is supplemented.

### (Supplementary Note 15)

The computer-readable recording medium according to Supplementary Note 13, in which
the biometric information includes time-series information acquired along a time series from each of a plurality of persons.

### (Supplementary Note 16)

The computer-readable recording medium according to Supplementary Note 15, in which
information indicating presence or absence of occurrence of a disease in the person includes presence or absence of occurrence of the disease for each elapsed time.

### (Supplementary Note 17)

The computer-readable recording medium according to Supplementary Note 13, in which
the disease is heart failure, renal failure, cerebral infarction, or diabetes.

### (Supplementary Note 18)

A computer-readable recording medium having recorded therein a program including commands for causing a computer to execute
an information acquisition step for acquiring biometric information of a person to be predicted, and
a disease prediction step for inputting the acquired biometric information to a prediction model and predicting presence or absence of occurrence of a disease in the person to be predicted from an output result of the prediction model,
in which the prediction model is generated by
generating, from a data group including biometric information of a person and information indicating presence or absence of occurrence of a disease in the person, a graph including nodes representing data points and edges representing a relationship between nodes,
supplementing a deficiency of the generated graph,
generating a data group in which a deficiency is supplemented from the supplemented graph, and
performing machine learning using the generated data group as training data.

While the present invention has been particularly shown and described with reference to example embodiments thereof, the present invention is not limited to these example embodiments. It will be understood by those of ordinary skill in the art that various changes in form and details may be made therein without departing from the spirit and scope of the present invention as defined by the claims.

This application is based upon and claims the benefit of priority from Japanese patent application No. 2023-026656, filed on February 22, 2023, the disclosure of which is incorporated herein in its entirety by reference.

### Industrial Applicability

As described above, according to the present disclosure, even in a case where a deficiency exists in training data of a prediction model for predicting a disease, prediction accuracy of the disease can be improved. The present disclosure is useful in a variety of systems relating to the diagnosis of diseases.

### Reference Signs List

- 10: learning model generation apparatus
- 11: graph generation unit
- 12: graph supplementation unit
- 13: model generation unit
- 20: prediction model
- 30: graph generation model
- 31: graph
- 32: node
- 33: edge
- 40: graph supplementation model
- 50: disease prediction apparatus
- 51: information acquisition unit
- 52: disease prediction unit
- 110: computer
- 111: CPU
- 112: main memory
- 113: storage device
- 114: input interface
- 115: display controller
- 116: data reader/writer
- 117: communication interface
- 118: input device
- 119: display device
- 120: recording medium
- 121: bus

## Claims

1. A learning model generation apparatus comprising:
a graph generation means for generating, from a data group including biometric information of a person and information indicating presence or absence of occurrence of a disease in the person, a graph including nodes representing data points and edges representing a relationship between the nodes;
a graph supplementation means for supplementing a deficiency of the generated graph; and
a model generation means for generating, from the supplemented graph, a data group in which a deficiency is supplemented, performing machine learning using the generated data group as training data, and generating a prediction model for predicting occurrence of a disease in the person.

2. The learning model generation apparatus according to claim 1, wherein
the graph supplementation means supplements a deficiency of the generated graph using a machine learning model constructed by performing machine learning on a relationship between a graph in which some nodes are deficient and a graph in which a deficiency is supplemented.

3. The learning model generation apparatus according to claim 1, wherein
the biometric information includes time-series information acquired along a time series from each of a plurality of persons.

4. The learning model generation apparatus according to claim 3, wherein
information indicating presence or absence of occurrence of a disease in the person includes presence or absence of occurrence of the disease for each elapsed time.

5. The learning model generation apparatus according to claim 1, wherein
the disease is heart failure, renal failure, cerebral infarction, or diabetes.

6. A disease prediction apparatus comprising:
an information acquisition means for acquiring biometric information of a person to be predicted; and
a disease prediction means for inputting the acquired biometric information to a prediction model and predicting presence or absence of occurrence of a disease in the person to be predicted from an output result of the prediction model,
wherein the prediction model is generated by:
generating, from a data group including biometric information of a person and information indicating presence or absence of occurrence of a disease in the person, a graph including nodes representing data points and edges representing a relationship between nodes;
supplementing a deficiency of the generated graph;
generating a data group in which a deficiency is supplemented from the supplemented graph; and
performing machine learning using the generated data group as training data.

7. A learning model generation method comprising:
generating, from a data group including biometric information of a person and information indicating presence or absence of occurrence of a disease in the person, a graph including nodes representing data points and edges representing a relationship between the nodes;
supplementing a deficiency of the generated graph; and
generating, from the supplemented graph, a data group in which a deficiency is supplemented, performing machine learning using the generated data group as training data, and generating a prediction model for predicting occurrence of a disease in the person.

8. The learning model generation method according to claim 7, wherein
the supplementing of the graph includes supplementing a deficiency of the generated graph using a machine learning model constructed by performing machine learning on a relationship between a graph in which some nodes are deficient and a graph in which a deficiency is supplemented.

9. The learning model generation method according to claim 7, wherein
the biometric information includes time-series information acquired along a time series from each of a plurality of persons.

10. The learning model generation method according to claim 9, wherein
information indicating presence or absence of occurrence of a disease in the person includes presence or absence of occurrence of the disease for each elapsed time.

11. The learning model generation method according to claim 7, wherein
the disease is heart failure, renal failure, cerebral infarction, or diabetes.

12. A disease prediction method comprising:
acquiring biometric information of a person to be predicted; and
inputting the acquired biometric information to a prediction model and predicting presence or absence of occurrence of a disease in the person to be predicted from an output result of the prediction model,
wherein the prediction model is generated by:
generating, from a data group including biometric information of a person and information indicating presence or absence of occurrence of a disease in the person, a graph including nodes representing data points and edges representing a relationship between nodes;
supplementing a deficiency of the generated graph;
generating a data group in which a deficiency is supplemented from the supplemented graph; and
performing machine learning using the generated data group as training data.

13. A computer-readable recording medium having recorded therein a program including commands for causing a computer to execute:
generating, from a data group including biometric information of a person and information indicating presence or absence of occurrence of a disease in the person, a graph including nodes representing data points and edges representing a relationship between the nodes;
supplementing a deficiency of the generated graph; and
generating, from the supplemented graph, a data group in which a deficiency is supplemented, performing machine learning using the generated data group as training data, and generating a prediction model for predicting occurrence of a disease in the person.

14. The computer-readable recording medium according to claim 13, wherein
the supplementing the graph includes supplementing a deficiency of the generated graph using a machine learning model constructed by performing machine learning on a relationship between a graph in which some nodes are deficient and a graph in which a deficiency is supplemented.

15. The computer-readable recording medium according to claim 13, wherein
the biometric information includes time-series information acquired along a time series from each of a plurality of persons.

16. The computer-readable recording medium according to claim 15, wherein
information indicating presence or absence of occurrence of a disease in the person includes presence or absence of occurrence of the disease for each elapsed time.

17. The computer-readable recording medium according to claim 13, wherein
the disease is heart failure, renal failure, cerebral infarction, or diabetes.

18. A computer-readable recording medium having recorded therein a program including commands for causing a computer to execute:
acquiring biometric information of a person to be predicted; and
inputting the acquired biometric information to a prediction model and predicting presence or absence of occurrence of a disease in the person to be predicted from an output result of the prediction model,
wherein the prediction model is generated by:
generating, from a data group including biometric information of a person and information indicating presence or absence of occurrence of a disease in the person, a graph including nodes representing data points and edges representing a relationship between nodes;
supplementing a deficiency of the generated graph;
generating a data group in which a deficiency is supplemented from the supplemented graph; and
performing machine learning using the generated data group as training data.
